# EUROPEAN PATENT APPLICATION

(11) **EP 4 461 284 A1**
(43) Date of publication of application: **13.11.2024**
(21) Application number: 23382436.6
(22) Date of filing: 10.05.2023
(51) Int. Cl.: A61H 9/00

(54) **APPARATUSES AND METHODS TO IMPROVE FUNCTIONING OF A PATIENT'S COLON**

(71) Applicant: Usmima, S.L., 08950 Esplugues de Llobregat (ES)
(72) Inventor: WILHEMS, Markus, 08015 Barcelona (ES); HERRERO FRESNEDA, Immaculada, 08758 Cervelló (ES); BENET POZO, Marc, 08960 Sant Just Desvern (ES); CALZADA DEFEZ, Angel, 08028 Barcelona (ES)
(74) Representative: Bardehle Pagenberg S.L.

(57) **Abstract**

The present disclosure is related to apparatuses configured to improve functioning of a patient's colon. The apparatuses comprise a support configured to be arranged at least partially around a waist of the patient and comprising a plurality of actuators configured to exert pressure against an abdominal wall of the patient. Further, the apparatuses comprise a control unit to control the actuators and sensing units configured to measure parameters associated with a condition of a section of a colon of a patient. Additionally, the control unit is in communication with the sensing units, and is further configured to determine a physiological condition in the section of the colon of the patient and to control the actuators at least partially based on the physiological condition. Methods and computer-implemented methods to improve functioning of a patient's colon and for determining a specific physiological condition in a section of the colon are also provided.

## Description

### FIELD

The present disclosure relates to apparatuses and methods configured to improve the functioning of a patient's colon.

### BACKGROUND

Chronic intestinal constipation is a common gastrointestinal disorder affecting a significant proportion of the population worldwide. It is a condition in which an individual experiences infrequent bowel movements or has difficulty passing stool, which can result in abdominal discomfort, bloating, and pain. Chronic constipation can significantly impact a person's quality of life, affecting their physical, mental, and social well-being.

Chronic intestinal constipation can be classified into primary or secondary, depending on its underlying cause. Primary chronic constipation is idiopathic, meaning there is no known underlying cause, and it is often characterized by slow transit, obstructed defecation, or a combination of both. Secondary chronic constipation is caused by an underlying medical condition, such as irritable bowel syndrome (IBS), thyroid disorders, or neurological disorders, among others.

There are several factors that can contribute to the development of chronic constipation. These include a low-fiber diet, inadequate fluid intake, lack of physical activity, certain medications, and underlying medical conditions, among others.

The management of chronic constipation may involve a multi-faceted approach, which may include lifestyle modifications, such as increasing fiber intake, drinking more fluids, and increasing physical activity (for those patients that can do so), as well as the use of laxatives or other medications. In severe cases, surgical interventions may be required.

In recent years, there has been a growing interest in developing new therapies and apparatuses for treating chronic constipation. Some of these therapies and apparatuses aim to improve gastrointestinal motility, increase secretion in the intestines, and improve the signaling of the enteric nervous system to promote bowel movements via stimulation, either mechanically or pharmaceutically, of mechanosensory cells, their circuits and neurotransmitters such as serotonin. One such approach is the automation of colon massage therapy to physically move the feces and stimulate the gut.

WO2017/042599 A1 discloses an abdominal massage apparatus having massage units, wherein massaging heads are arranged such that they are adjacent to at least one section out of an ascending section, transverse section or descending section of the colon of the patient. Specific treatment frequencies and treatment programs are disclosed. Although the massage apparatus and methods disclosed in this prior art document have important advantages over other devices, further improvements are still possible.

It has been found that methods and apparatuses such as the ones disclosed in WO 2017/042599 can also improve the functioning of the colon for other patients. For example, patients that have irritable bowel syndrome, celiac disease or inflammatory bowel disease can have either constipation symptoms or frequent or chronic diarrhea, or both.

The present disclosure provides improved apparatuses and methods for stimulating colon activity in case of constipation or other bowel dysfunctions and for improving colon functioning in general.

### SUMMARY

In a first aspect of the present disclosure, an apparatus to improve functioning of a patient's colon is provided. The apparatus comprises a support configured to be arranged at least partially around a waist of the patient. The support comprises a plurality of actuators configured to exert pressure against an abdominal wall of the patient. The apparatus further comprises a control unit configured to control the actuators. Further, the apparatus comprises one or more sensing units configured to measure one or more parameters associated with a condition of at least a portion of a colon of a patient. Further, the apparatus comprises a control unit in communication with the sensing units. The control unit is configured to determine a physiological condition in the section of the colon of the patient and to control the actuators at least partially based on the physiological condition.

According to this aspect, an apparatus is provided which can improve colon activity by adapting a treatment to specific condition in the colon and/or to a specific patient, i.e. by personalizing the treatment. The apparatus may determine the portion of the colon wherein actuation may be required based on the measurements performed, and may adapt its operation accordingly. Further, the fact that the apparatus determines a physiological condition in a portion of the colon may allow stimulating specific sensory circuit in specific regions or sections of the colon, which can promote peristalsis and facilitate the movement of stool through the colon. This can help to reduce the time it takes to pass stool and reduce the discomfort associated with constipation.

Thus, the use of the disclosed apparatus can help to improve the quality of life of individuals suffering from (chronic) constipation by providing a safe, non-invasive, and effective way to promote bowel movements, particularly regular and easy bowel movements. By improving peristalsis, individuals with (chronic) constipation can experience greater comfort, reduced reliance on medications, and improved overall health and well-being.

Throughout the present disclosure, an actuator may be regarded as any part or component that transmits force or energy to the abdomen of the patient, either directly or indirectly. In particular, the actuators may rely on converting electrical, pneumatic or hydraulic energy into mechanical force and/or movement.

Improving the functioning of the patient's colon as used throughout the present disclosure is to be interpreted as covering stimulating a patient's colon to reduce or avoid constipation as well as avoiding or reducing diarrhea, and to cover reducing bowel dysfunction in general, including e.g. irritable bowel syndrome, inflammatory bowel diseases, fecal incontinence, chronic constipation, chronic diarrhea and others.

In a further aspect, the present disclosure provides a method for treating a patient. The method comprises determining a physiological condition in at least a section of the colon. Further, the method comprises a plurality of actuators applying a localized pressure against the abdominal wall in order to stimulate colon activity/evacuation. Additionally, the actuators apply the localized pressure at least partially based on the determined physiological condition.

In yet a further aspect, the present disclosure provides a computer-implemented method for treating a patient. The method comprises receiving data indicative of a physiological condition in at least a section of a colon of the patient from one or more sensing units and determining a physiological condition in at least a section of the colon. The method further comprises controlling a plurality of actuators applying a localized pressure against the abdominal wall in order to stimulate colon activity/evacuation, wherein the actuators apply the localized pressure at least partially based on the determined physiological condition.

Controlling the actuators herein refers to sending control signals to the actuators themselves or related drive or control mechanisms such that the actuators executed a determined treatment. Such a method may be fully computer-implemented and executed by a control unit which may form part of or be connected to examples of the apparatus to improve functioning of a patient's colon of the first aspect.

Throughout this disclosure, the term "abdominal wall" may be understood as the abdominal skin facing a substantially anterior side, i.e. ventral side, of the patient's body. The term "physiological condition" may be understood as any a condition or state of the colon or a section of the colon. The physiological condition may particularly refer to a condition that deviates from a standard or norm, and relate to e.g. physiological abnormalities or pathology. A physiological condition of the colon may include the presence of fecalomas, or hardening of internal organs among others. In further examples, the physiological condition may refer to volvulus (an intestinal obstruction), bloating, hardening, presence of fecal mass, movement patterns of the colon, abdominal distension and others. The term "physiological abnormality" may be understood as an abnormal condition experienced in a body organ or system, particularly in the colon or section of the colon.

Additional objects, advantages and features of embodiments of the present disclosure will become apparent to those skilled in the art upon examination of the description, or may be learned by practice.

### BRIEF DESCRIPTION OF THE DRAWINGS

Figures 1A and 1B schematically illustrate a perspective view of two different examples of an apparatus to improve the functioning of a patient's colon;
Figures 2A and 2B schematically illustrate perspective views of an example of a base unit comprising a control unit of an apparatus according to an example of the present disclosure;
Figures 3A - 3C schematically illustrate examples of arrangements of actuators with respect to a patient's colon;
Figures 4A and 4B schematically illustrate further examples of apparatus for improving functioning of the colon;
Figures 5A - 5C schematically illustrate further examples of an apparatus for improving functioning of the colon;
Figure 6 is a graph illustrating an example of the evolution of pressure measured by a sensing unit as a function of time, in one implementation;
Figures 7A and 7B show flow diagrams of examples of methods for treating a patient according to examples of the present disclosure; and
Figures 8A and 8B schematically illustrate further examples of methods according to the present disclosure.

### DETAILED DESCRIPTION OF EXAMPLES

Reference will now be made in detail to embodiments of the disclosure, one or more examples of which are illustrated in the drawings. Each example is provided by way of explanation, not as a limitation. In fact, it will be apparent to those skilled in the art that various modifications and variations can be made in the present disclosure without departing from the scope or spirit of the teaching. For instance, features illustrated or described as part of one embodiment can be used with another embodiment to yield a still further embodiment. Thus, it is intended that the present disclosure covers such modifications and variations as come within the scope of the appended claims and their equivalents.

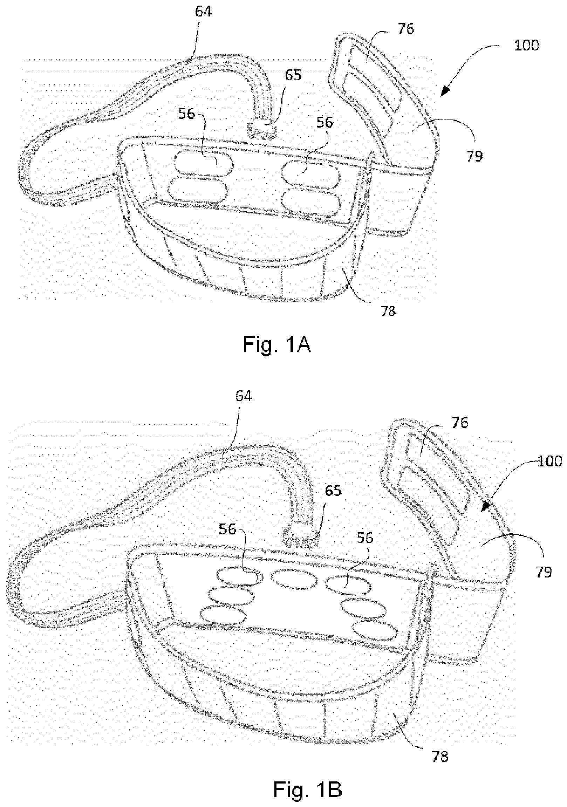

Figure 1A is a perspective view of an example of an apparatus 100 to improve the functioning of a patient's colon. The apparatus 100 might be used to reduce or avoid constipation The apparatus 100 comprises a support 78 configured to be arranged at least partially around a waist of the patient. The apparatus 100 comprises a plurality of actuators 56 configured to exert pressure against an abdominal wall of the patient. Additionally, the apparatus 100 comprises a control unit (not shown) configured to control de actuators 56. Further, the apparatus also includes one or more sensing units configured to measure one or more parameters associated with a condition of at least a portion of a colon of a patient. In addition, the control unit is in communication with the sensing units, and is further configured to determine a physiological condition in at least a section of the colon of the patient, and to control the actuators 56 at least partially based on the physiological condition.

In the example of figure 1A, the support 78 is a belt which can be easily secured to the patient's abdomen. In this example, the belt substantially surrounds the abdomen of the patient, but in other cases, the actuators 56 may be located in a support 78 that may be at least partially open. For example, the support 78 may have an open section configured to be placed at the lumbar region of the patient, i.e. the support 78 may not fully surround the abdomen of the patient.

As shown in figure 1A, the belt 78 may comprise one or more straps 79, or any other suitable mechanism to adjust the perimeter of the same around the abdomen of the patient. The strap 79 may include patches 76 of a hook-and-loop fastener like Velcro^{™}. Other closing mechanisms including buckles, straps and others may be adjust to fit and adjust the belt 78 around a patient's waist.

Thus, when the support 78 (with the actuators 56) is secured to the patient's abdomen, the operation of the actuators 56 may generate a compressive force against the abdomen of the patient. In the specific example illustrated in figure 1A, four actuators 56 are provided. These are arranged such that when the belt is fitted around the patient's waist, they are arranged on top of or aligned with sections of the colon. In some examples, a patient may wear the belt which completely surrounds the patient's waist. In other examples, the belt may be attached to a bed or a wheelchair and may be arranged such that the actuators of the belt are in a position to exert a pressure on the patient's abdomen.

In the example in figure 1A, the apparatus further includes conduits 64 between the actuators 56 and a fluid supply (not illustrated). The conduits 64 may be embodied as flexible hoses. The conduits 64 may have a suitable connector 65 at their end to connect to a fluid supply. The fluid supply may be a pump or a fluid supply line, e.g. pressurized conduit of liquid or gas. In the example in figure 1, the sensing units comprise pressure sensors which may be located at any point along the conduits 64, adjacent to/at the actuators 56, or at the exit of the pump among other locations.

The control unit may also comprise one or more pressure regulators to adapt the fluid pressure reaching the actuators 56. In addition, the apparatus may further comprise one or more electrovalves in fluid communication with the actuators 56. Thus, a single pressurized line or pump may be used to feed several actuators 56 independently. In other examples, different control elements and/or multiple pumps may be used to control individual or groups of actuators.

In specific methods of treatment using the example of figure 1, the actuators may be inflated to a given pressure level and then deflated. Such a pressure level may be e.g. 375-525 mmHg.

Figure 1B discloses a similar example of an apparatus 100. The main difference between the example of figure 1B and the example of figure 1A is that the number of actuators 56 and their positions with respect to patient's colon (once the belt is worn by the patient) are slightly different.

In some examples, at least one of the parameters measured by the sensing units of apparatus 100 may be an operational parameter of the actuators 56, e.g. operating pressure, actuator displacement, actuator acceleration, or other. The values of the parameters measured by the sensing units may be used to determine or estimate a physiological condition of the patient's colon. For example, the operating pressure of the actuators 56 may be used to identify whether a section of the colon of the patient is at least partially obstructed.

In other examples, the sensing units may comprise a resistivity sensor, a salinity sensor, an ultrasonic sensor, a microphone or other sound amplifier (including e.g. stethoscope), or other types of sensors that may be used to obtain parameters that may be indicative of or used to determine or estimate a physiological abnormality of the colon of the patient. More precisely, resistivity sensors and/or salinity sensors may be configured to be in contact with the skin of the patient to measure variation in resistivity at different locations of the epidermis of the patient. An ultrasonic sensor may be included in the apparatus to aid in the visual inspection of the colon of the patient. Additionally, one or more microphones may be included in the apparatus to record sounds from the colon, e.g. to monitor how sound propagates when the abdomen of the patient is gently tapped. Alternatively microphones or audio devices may be used to assess bowel sounds and/or murmurs.

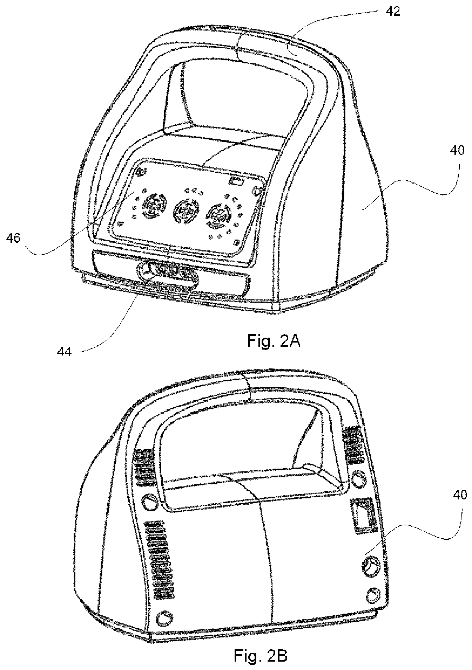

Figures 2A and 2B provide a front and rear view of a base 40 which may be used in combination with an apparatus 100 as shown in the examples of figure 1. In this example, the control unit is located in the base 40 and is thus separate from the support 78 and actuators 56. In other examples, the control unit may be included in the belt 78 as will be illustrated herein.

The base 40 may include a suitable housing, a power supply, a fluid supply. In this particular case, the processes carried out by the apparatus 100 may be controlled by the base 40 since the control unit is located inside the base 40.

In this example, the base 40 further includes a handle 42 such that the base 40 is portable and may be carried by a patient or e.g. a caretaker. The base 40 may further include a user interface 46 through which a user may interact with the apparatus 100 and base 40. The user interface 46 may include or may be e.g. a touch screen display, a plurality of knobs, handles or switches to control functions of the apparatus 100.

In particular, a patient may select a suitable treatment program, or treatment time, or may activate (i.e. start) or deactivate (i.e. interrupt or stop) a treatment. The user interface 46 in some examples may further provide feedback or information to a user e.g. about progress of the treatment, duration thereof, intensity thereof etc.

The control unit may be configured to command the sensing units, the actuators 56, and other associated components, such as e.g. the electrovalves and the pump in the present example (and other actuators in other examples).

The control unit may include a processor configured to perform the methods and/or steps described herein. Further, in some examples, the sensing units may also include a processor.

As used herein, the term "processor" is not limited to integrated circuits referred to in the art as a computer, but broadly refers to a controller, a microcontroller, a microcomputer, a programmable logic controller (PLC), an application-specific, integrated circuit, and other programmable circuits, and these terms are used interchangeably herein. It should be understood that a processor and/or a control system can also include memory, input channels, and/or output channels.

Examples of the methods disclosed herein may be implemented with hardware, software, firmware and combinations thereof. Examples of the methods disclosed herein may be implemented using artificial intelligence and/or algorithms derived from a process of machine learning.

In the example of figure 2 the housing of the base 40 comprise a plurality of ports 66 configured to be coupled with the respective connectors 65 of the conduits 64 (see figure 1). In the example of figure 2, these ports 66 may also be connected to the sensing units, which may be included in the housing of the base 40. In some examples, the sensing units may measure an operation time of the actuators 56 until at least one of the parameters measured by the sensing units reaches a threshold. For example, the sensing units may measure how long it takes to reach a certain level of pressure inside the conduits 64, in particular when fluid activated actuators are used. A time that it takes to reach a certain pressure level can be indicative of an obstruction in the colon.

In other examples, the sensing units may be located next to the actuators 56 or e.g. inside fluid chambers associated with the actuators 56, and the conduits 64 may also comprise electrical connections to connect the sensing units with the control unit 68. Other connections, such as a wireless connection e.g. Bluetooth, WiFi or Zigbee. As previously discussed, the time taken for the actuators 56 to reach an operating pressure may be used to estimate a condition of a section of the colon of the patient.

Further, the control unit may be configured to determine whether a portion of the colon is subjected to physiological abnormalities at least partially based on the measured operation time of the actuators 56 and/or on a difference in operation time compared to different actuators 56. In examples, the control unit may determine a deviation of the operation time of an actuator 56 from a mean operation time of two or more actuators 56. The magnitude of the deviation may be used to determine a physiological condition, e.g. a physiological abnormality. In some examples, the measured operation time may be compared with a reference value. Such as a reference value may be based on average values determined for a baseline population, or average values for a population with the same or similar the body mass index of the patient. Reference values may also be based on historical data of the same patient, or data obtained from different sections of the colon of the patient. The same approach to determine a physiological condition may be used when determination is based on other measured parameters (skin resistivity, salinity, sound etc.)

In some examples, the control unit may record a signal comprising one of the measured parameters as a function of time. This signal may be processed by the control unit 68 to obtain time-dependent information associated with the actuators 56 and patient. For example, the recorded signal may be processed to estimate the dominant frequencies of the signal, e.g. a pressure signal. These computed parameters may be later compared with reference values to determine a condition of a portion of the colon of the patient. The reference values may again be based on data of the general population, historical data of the same patient etc.

In other examples, the sensing unit may measure the velocity of the actuators 56 for a given pressure increase, e.g. using accelerometers. These other parameters may be used to estimate whether a portion of the colon is subjected to physiological abnormalities and/or to determine a physiological condition of a portion of the colon.

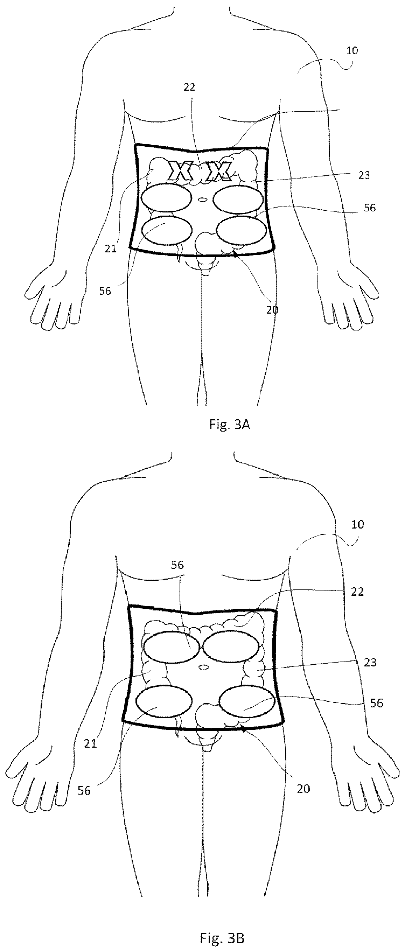

Figures 3A - 3C schematically illustrate different examples of arrangements of actuators 56 which may be used in examples of the apparatus 100 i.e. different arrangements of actuators 56 that may be provided in a belt 78 or other support.

Figure 3A illustrates a subject or patient 10 is illustrated. The subject has a colon 20, including an ascending section 21, a transverse section 22, and a descending section 23. In the example of figure 3A, the actuators 56 are arranged in two pairs: two actuators are substantially arranged along the ascending section 21, and two actuators 56 are arranged along the descending section 23. In the example of figure 3A, as schematically illustrated with crosses, no actuators are arranged on or aligned with the transverse section. It has been found that the position of the transverse section in different patients may vary more than the positions of the ascending and the descending sections which are quite uniform throughout the population. Also, actuating on the transverse section may, in some patients, involving exerting pressure on the patient's diaphragm and/or on a patient's stomach. This may be unpleasant for patients and (in some patients) affect the patient's breathing.

Figure 3B illustrates a different configuration of the actuators 56. Also in this example, four actuators are provided. In this case, the upper actuators are arranged such that in use they are positioned substantially on top of the transition between the ascending and transverse section and the descending and transverse sections respectively.

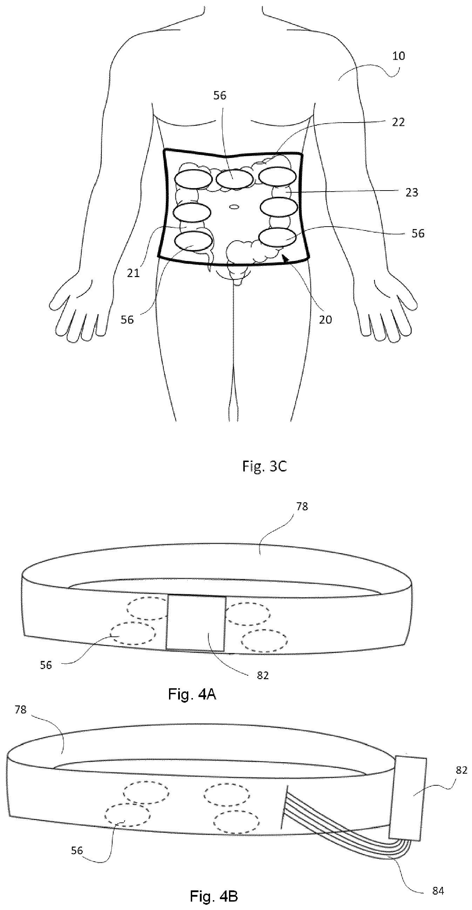

Figure 3C illustrates yet a further example which may be used: The apparatus 100 in this example comprises a plurality of actuators 56 arranged such that they describe an inverted U. Thus, some actuators 56 may be located substantially adjacent to an ascending section of the colon, some other actuators 56 adjacent to a descending section of the colon and other actuators 56 adjacent to a transverse section of the colon of the patient. The example in figure 3C comprises seven actuators 56.

It will be clear however that other numbers and distributions of actuators are also possible. For example, the apparatus 100 may comprise one, two or more actuators 56 at each lateral segment of the inverted U, i.e. corresponding to the ascending and the descending portion of the colon; and none at the upper segment of the inverted U, i.e. corresponding to the transverse portion of the colon.

The actuators 56 in the examples of figures 3A - 3C are inflatable actuators, but linear actuators, rotary actuators, and others may be also included depending on the type of stimulation that it is to be achieved at the respective colon portion. Further, the type and dimensions of the actuators may also be selected based on patient size and e.g. on the characteristics of the pressure pattern/sequence to apply. For example, a rotary actuator, such as mechanisms comprising cams and others, may be suitable for operation sequences with high frequency of operation, whereas a linear actuator may be suitable for operation sequences for which high precision in volume displacement may be required.

In each of the examples illustrated hereinbefore, the control unit 68 of the apparatus 100 may be configured to control the actuators 56 such that a cyclic actuation with a specific frequency is provided. A frequency range of up to 0.9 Hz, specifically of 0.1 - 0.9 Hz has been found to be particularly suitable for the majority of patients as the frequency range coincides/overlaps with the contraction frequency of a healthy colon.

The control unit 68 may be configured to control the actuators 56 (and other components of the apparatus 100) such that at least a plurality of actuators 56 are operated simultaneously, i.e. inflated or otherwise extended at the same time.

Furthermore, the control unit 68 may also be configured to control the actuators 56 such that a specific order or sequence in actuation is followed. This sequence may include an actuator operating pressure that decreases in magnitude consecutively from the abdominal surface adjacent to the base of the ascending colon towards the abdominal surface adjacent to the descending colon. Additionally, the sequence of actuation may include an actuator operating pressure that increases in magnitude consecutively from the abdominal surface adjacent to the base of the ascending colon towards the abdominal surface adjacent to the descending colon.

Figures 4A and 4B schematically illustrate other examples of an apparatus for improving or promoting functioning of the colon. The apparatus disclosed is generally similar as the apparatus disclosed in figure 1. However, in this case, a control unit 82 is integrated in the support or belt 78 which also carries actuators 56. The control unit 82 may be responsible for the control of the actuators 56 and sensing units (not shown) etc. as described hereinbefore with reference to figures 1 and 2 but it is integrated in the support rather than in the base. The base may be dispensed with (depending on how actuators are implemented) or simplified. In examples, the user interface may be integrated in the control unit 82 or in the support 78.

Figure 4B illustrates yet another example, in which the control unit is not integrated in or carried by support 78. Rather, the control unit 82 is connected through cables 84 with support 78. The cables may carry control signals from control unit 82 towards the actuators and receive signals regarding measurements from sensor units. Similarly, as to the example of figure 4A, the base illustrated in figure 2 may be dispensed with or may be simplified.

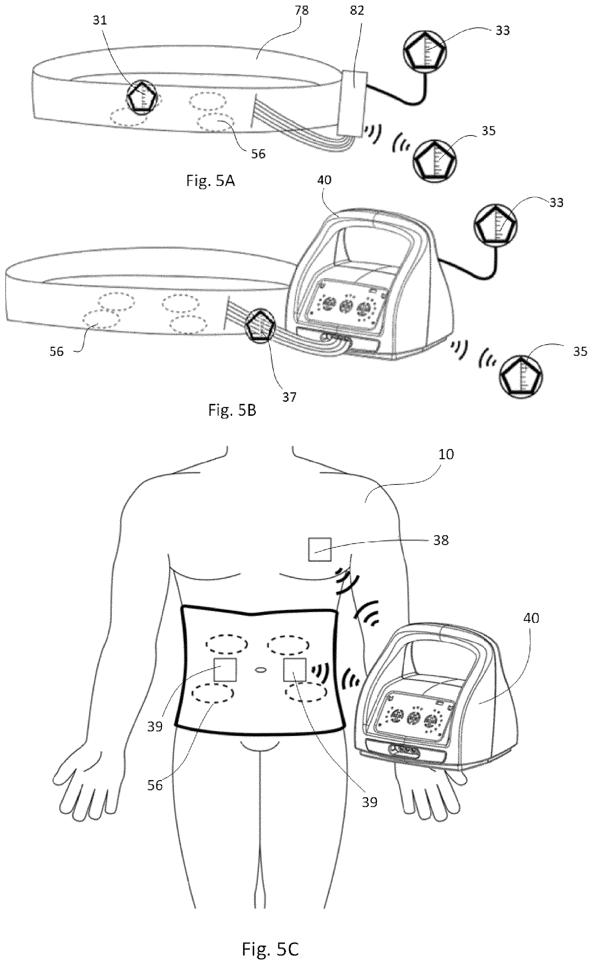

Figures 5A and 5B illustrate other examples of an apparatus 100 according to an example of the present disclosure. In the example of figure 5A, the belt 78 comprises four actuators 56. The control unit 82 in this example is connected through cables to the belt 78. In the example of figure 5A, different sensing units may be used.

One or more sensing units 31 may be arranged with one or more of the actuators 56. The sensing unit 31 may e.g. be a pressure sensor, particularly in case of inflatable or fluid driven actuators. In other examples, sensors may measure the incursion or movement or speed or acceleration of the corresponding actuator.

In the example of figure 5A, one or more further sensing units 33 may be arranged with the control unit 82. The sensing unit 33 may also be a pressure sensor in case of an inflatable actuator. Rather than measuring the pressure at the actuator 56, the pressure may be measured elsewhere in the system. Other suitable sensing units 33 may also be arranged with the control unit 82.

Further, as shown in the example of figure 5A, a further separate sensing unit 35 which may have a wireless connection with the control unit 82 may be used as well. The sensing unit 35 or the sensing unit 33 in figure 5A may be a probe, e.g. an ultrasound probe. In this example, the probe may be connected to the control unit 82, which at the same time, may be connected to a peripheral device, e.g. a screen, to allow visual inspection of the colon of the patient. The ultrasound probe may be used prior to treatment (e.g. to determine physiological condition in the colon), during treatment and/or after treatment (to determine effectiveness of the chosen parameters).

In other examples, the sensing unit 35 of the apparatus 100 may include a radiography apparatus, a computerized tomography scanner or a magnetic resonance imaging apparatus among others. These apparatuses herein disclosed may be combined with other sensing components, such as pressure sensors, to verity the patient condition and/or monitor the condition of the patient during treatment.

In further examples, auscultation with a stethoscope or similar might be used. Bowel sounds may be characterized as absent, normoactive, hypoactive, or hyperactive. Absent bowel sounds may indicate ileus or peritonitis but may also be indicative of volvulus or any intestinal obstruction. Hyperactive bowel sounds may occur with an early intestinal obstruction or gastrointestinal hypermotility. Sluggish gut sounds may indicate an obstruction.

Although the measurements taken by these sensing units may generally be inspected by a medical professional to determine a condition of the patient, the control unit of the apparatus 100 of the present disclosure may comprise an artificial intelligence module configured to inspect the measurements, and also configured to provide an estimation of a condition of the patient.

In the example of figure 5B, the apparatus 100 may include belt 78 and base 40, which incorporates the control unit. In the example of figure 5B, one or more sensing units 37 are shown to be arranged with the tubes connecting the belt 78 to the base which can supply air to and from the actuators. Also, one or more sensing units 33 may be connected with the control unit in the base 40, and one or more separate sensing units 35 with a wireless connection may also be used.

Figure 5C illustrates yet a further example. In this example, the support or belt may carry again four actuators. Sensing units 39 in this case may be arranged with the support or belt, but not directly with the actuators. Further sensing units 38 may be arranged on the subject 10 and may be included e.g. in an adhesive patch. The sensing units 39 and/or sensing units 38 may have a wireless connection with the control unit, which in this case may be incorporated in base 40.

The sensing unit 39 and/or the sensing unit 38 may e.g. be a skin resistivity sensor, an ultrasonic sensor, a microphone or other sound amplifiers or audio device (including e.g. stethoscope).

It should be clear that the arrangement of sensing units of any of figures 5A - 5C may be combined with any of the hardware configurations of figures 5A - 5C.

In examples of the present disclosure, an apparatus may be embodied as a kit of parts, i.e. the apparatus may comprise a belt with a plurality of actuators, and one or more separate sensing units e.g. stick-on patches for ultrasound and/or for microphones/stethoscopes. Such separate sensing units may include wireless communication capabilities for communicating with the control unit.

In any of the examples disclosed herein, a method of treatment may include a first phase of "diagnosis" or mapping of a situation in the colon. Then, the treatment parameters may be adapted at least partially based on the diagnosis. Parameters such as frequency, pressure, sequence of actuation, inflation rate in case of pneumatic or hydraulic actuators and others may be adapted on the basis of the diagnosis.

Such a diagnosis may be provided at the beginning of a treatment to determine treatment parameters. In some examples, it may be repeated during the treatment e.g. to determine whether the situation has improved or changed. If the physiological condition in the colon or sections of the colon has changed, then treatment parameters may be adapted again. Finally, a diagnosis or mapping may be repeated after a treatment as well to determine the overall efficiency and/or effectiveness of the treatment.

In some examples, treatment parameters and determined effectiveness thereof can be stored to determine which treatment parameters are effective for a specific condition and/or for a specific patient.

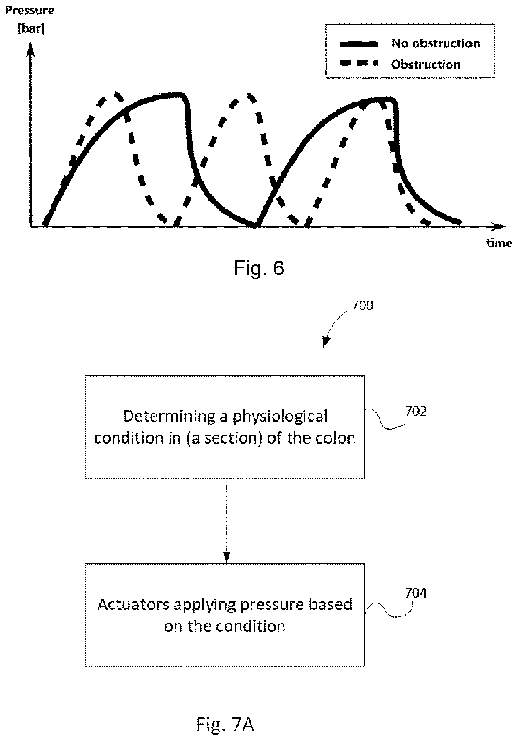

Figure 6 shows a graph illustrating an example of the evolution of pressure measured by a sensing unit as a function of time. Figure 6 includes two different lines, a first line corresponds to a pressure evolution measured by a pressure sensor associated with an actuator acting over a healthy and/or unobstructed portion of the colon; and a second line corresponding to a pressure evolution measured by a pressure sensor associated with an actuator acting over a portion of the colon in which a fecaloma is present.

As previously discussed, pressure signals may be recorded and processed to quantify the differences in response between portions of the colon of the patient, or between a portion (or all) of the colon relative to a reference value.

It may be seen in figure 6, that the pressure increases more rapidly for the actuator that is located substantially on top of the fecaloma. The fecaloma is a hard obstruction which causes the rapid pressure increase.

One way to determine the presence of an obstruction like a fecaloma may be to inflate all actuators (sequentially or simultaneously), possibly multiple times. Sensors may be used to determine the time it takes for each of the actuators to reach a specific pressure level. A mean or average time to reach the pressure level may be determined. Any of the actuators that takes significantly shorter or longer to reach the pressure level may be positioned on a section of an abnormality. Particularly, in the case of an obstruction, the pressure level may be reached rapidly.

As mentioned before, the diagnosis phase may be repeated multiple times.

An advantage of using an average or mean of the time that it takes to reach a specific pressure level is that the tightness of the belt (which may be adjusted by the user him/herself) will affect the pressure level in all actuators, so that a potential influence of the tightness of the belt is discarded, since only specific actuators will deviate from others. In alternative example, a sensor system may be implemented to determine the tightness and thereby take this into account in the measurements and/or treatment.

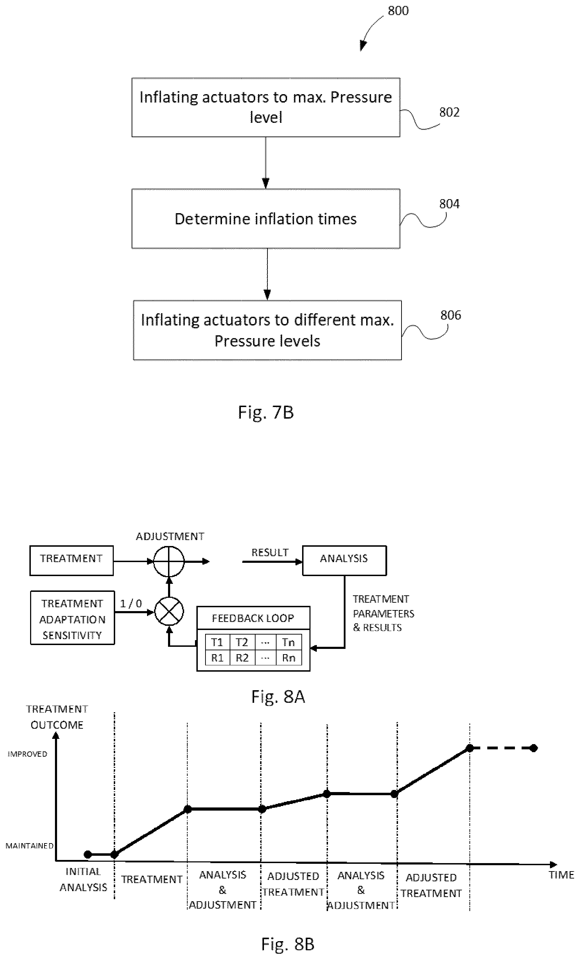

Figure 7A shows a flowchart of an example of a method 700 for treating a patient according to the present disclosure. The method 700 in figure 7 comprises, at block 702, determining a physiological condition in at least a section of the colon. The method 700 also comprises, at block 704, a plurality of actuators applying a localized pressure against the abdominal wall in order to stimulate colon activity or proper functioning of the colon. Further, the actuators apply the localized pressure at least partially based on the diagnosed physiological condition.

As previously discussed, method 700 can identify a condition of a colon of the patient, e.g. a physiological abnormality; and therefore, the operation of the actuators 56 may be adapted accordingly.

In some examples, applying a localized pressure against the abdominal wall of the patient includes operating more than one actuator 56 following a sequence of operation, e.g. a synchronized sequence of operation. The sequence of actuation may comprise a decreasing operating pressure in consecutive actuators 56 from the abdominal surface adjacent to the ascending portion 21 of the colon towards the abdominal surface adjacent to the descending portion 23 of the colon. In other examples, the sequence of actuation may comprise an increasing operating pressure in consecutive actuators 56 from the abdominal surface adjacent to the ascending portion 21 of the colon towards the abdominal surface adjacent to the descending portion 23 of the colon.

In other examples, applying a localized pressure against an abdominal wall of the patient is performed by operating the actuators 56 simultaneously. For example, the actuators 56 at each lateral side of the inverted U in the example in figure 3 may be operated simultaneously.

When e.g. a fecaloma has been diagnosed, the treatment parameters may be adjusted in a variety of ways. When another type of obstruction has been detected, treatment may be interrupted completely.

In some examples, applying a localized pressure may comprise adjusting the actuators 56 located upstream of the section of the colon where a fecaloma has been diagnosed. The upstream actuators 56 may apply a greater pressure than the average operating pressure of the remaining actuators 56. Further, the upstream actuators 56 may maintain the applied pressure for a longer period than the remaining actuators 56. In some cases, the upstream actuators 56 may increase the operating pressure at a low rate or at a relatively high rate.

Alternatively or additionally, applying a localized pressure may comprise adjusting the actuators 56 located downstream of the section of the colon wherein an obstruction has been diagnosed. The downstream actuators 56 may apply a lower pressure than the average operating pressure of the remaining actuators 56. Further, the downstream actuators 56 may maintain the applied pressure for a shorter period than the remaining actuators 56. In some cases, the downstream actuators may increase the operating pressure at a low rate or at a relatively high rate.

Additionally or alternatively, applying a localized pressure may comprise adjusting the actuators 56 substantially position on top of the section of the colon wherein an obstruction has been diagnosed. Thus, the actuators 56 may apply a localized pressure substantially over the section of the colon e.g. loaded with feces. The actuators 56 may apply a repetitive sequence of changing pressure magnitude. For example, the actuators may apply a repetitive sequence of high pressure and low pressure to produce an oscillatory motion of the abdominal wall of the patient. In other examples, the actuators position of top of the obstruction may not be inflated at all.

In further examples, applying 704 a localized pressure may comprise operating a plurality of actuators 56 simultaneously to improve overall intra-abdominal pressure. This may aid the evacuation of the colon. In examples all actuators may be inflated and deflated at a frequency that aims to cause resonance and thereby movement of matter in the colon.

Further, the aforementioned examples may be combined during treatment of a patient.

A further example of a method of treatment 800 is illustrated in figure 7B. In some examples, diagnosing a physiological condition in at least a section of the colon includes operating one or more actuators 56 that apply a localized pressure against the abdominal wall of the patient. Then, measuring one or more parameters associated with a condition of a portion of a colon 20 of a patient, and processing the measured parameters to identify the portion of the colon with a physiological condition.

In examples, the method 800 includes recording and/or monitoring a pressure exerted by the actuators 56 as a function of time. Further, processing the measured parameters includes determining an operation time of the actuators 56 until reaching an operating pressure threshold, at block 802. Specifically, as mentioned before, actuators may be controlled in normal operation to reach a specific threshold of excursion (e.g. a mechanical extension or inflation to a specific level). Since actuators are controlled based on this principle, the same method may advantageously be used to determine a physiological condition in (a section of) the colon. After determining the inflation times, at block 804, of individual actuators, the data may be processed. At block 806, the inflation of the actuators may be adjusted, e.g. operational pressure levels may be defined for individual actuators.

In further examples, processing the measured parameters may include computing a difference between the recorded signal and a reference signal. In further examples, processing the measured parameters includes estimating the frequency content of the recorded signal. As previously discussed in relation with the control unit of the apparatus 100, the dominant frequency of the recorded signal may be used as an indicator for physiological abnormality. Additionally, a combination of measured parameters may be used to determine whether a portion of a colon of a patient is subjected to physiological abnormality, and that these may be combined with personal information of the patient, e.g. body mass index, anatomical abnormalities, positioning of ostomy or implants etc. for the same purposes.

In some examples of method 700, determining 702 a physiological condition in at least a section of the colon includes at least one of performing an ultrasonic scan, a planar radiography, a computerized tomography scan or a magnetic resonance imaging of the abdomen of the patient.

Further, the aforementioned techniques to diagnose or determine a physiological condition in a section of the colon may be combined with other techniques such as the application of localized pressure, as previously discussed. For example, in some examples, the method may include identifying a physiological condition in a section of the colon by applying the steps in relation to the application of localized pressure; and later perform any of the other techniques. Thus, a more detailed diagnosis may be achieved.

Figures 8A and 8B show examples of how the determination of a physiological condition in the colon or sections of the colon may be used to adjust operational parameters.

Prior to starting of the treatment, the apparatus may generate the treatment session to be applied to the patient. The selection may be based on patient data (age, sex, weight, clinical history) and/or on data obtained by the sensing units.

Theoretically the effects of the treatment should cause improvements on the patient's condition or status. The status of the patient's condition after having received some treatment (which can be a few seconds or few minutes after beginning the treatment session) is considered a result. This result ideally should differ from his/her status before initiating the session treatment. The degree of improvement (or even worsening) can be related to the treatment variables chosen. The result is analysed together with the treatment parameters used in the previous time period.

This information can be used as feedback to adjust the treatment for the subsequent time period. There may be a feedback buffer which allows to take into account historical data of the treatment, and adaptations performed and the results obtained. Each pair of treatment adjustment and result obtained may be understood as a feedback sample. The amount of feedback samples to be taken into consideration for the treatment adjustment can be tuned as well depending of the speed of adaptation and sensitivity desired.

In the specific example of figure 8A, the feedback branch can be enabled or disabled, which would prevent the treatment adjustments and keep the treatment constant.

Figure 8B is another illustration of how mapping of the status of the colon, or determination of conditions of sections of the colon may be carried out prior to or at the beginning of the treatment. The initial operational parameters may therefrom be derived and be at least partially based on the obtained data. Treatment may then be carried out during a first period of time, e.g. 5 - 20 minutes.

The mapping or determination may then be repeated to determine whether progress has been made and/or whether the physiological condition in one or more sections of the colon has changed. In such a case, the operational parameters may be adjusted and treatment may continue for a second period of time, e.g. 5-20 minutes. This sequence may be repeated throughout the treatment time. I.e. treatment may be interrupted for a determination or mapping phase multiple times until treatment is finished. The determination or mapping time periods may generally be shorter e.g. up to 5 minutes, specifically up to 3 minutes, than the treatment periods.

As mentioned elsewhere, the treatment data may be stored for later use. Particularly, the data may be stored such that the apparatus may determine suitable treatment parameters for future treatments.

It is noted that all features of the apparatus can be included in the methods for treating a patient, and *vice versa.*

For reasons of completeness, a number of aspects of the present disclosure are set out in the following numbered clauses:
Clause 1. An apparatus configured to improve functioning of a patient's colon, comprising:
   a support configured to be arranged at least partially around a waist of the patient and comprising one or more actuators configured to exert pressure against an abdominal wall of the patient;
   a control unit configured to control the actuators; and
   one or more sensing units configured to measure one or more parameters indicative of a condition of at least a section of a colon of a patient; wherein
   the control unit is in communication with the sensing units, and is further configured to determine a physiological condition in the section of the colon of the patient and to control the actuators at least partially based on the physiological condition.
Clause 2. The apparatus of clause 1, wherein the sensing units are configured to measure an operating pressure of the actuators, and the control unit is configured to measure an operation time of the actuators until the operating pressure reaches a threshold.
Clause 3. The apparatus of clause 2, wherein the control unit is configured to determine a physiological condition in a section of the colon at least partially based on the measured operation time of the actuators and/or on a difference in operation time associated with different actuators.
Clause 4. The apparatus of any one of clauses 1 - 3, wherein the control unit is configured to record a signal comprising at least one of the parameters measured by the sensing units as a function of time during operation of the actuators.
Clause 5. The apparatus of clause 4, wherein the control unit is configured to determine a physiological condition of a section of the colon at least partially based on a frequency content of the recorded signal.
Clause 6. The apparatus of any of clauses 1 - 5, wherein the control unit is configured to determine a physiological condition of a section of the colon at least partially based on a difference between a value of a parameter and a reference value.
Clause 7. The apparatus of any one of clauses 1 - 6, comprising two actuators arranged such that in use they are located adjacent to an ascending section of the colon and two actuators configured such that in use they are located adjacent to a descending section of the colon.
Clause 8. The apparatus of any one of clauses 1 - 7, wherein the sensing unit comprises pressure sensors.
Clause 9. The apparatus of any one of clauses 1 - 8, wherein the actuators include inflatable elements.
Clause 10. The apparatus of clause 9, wherein the inflatable elements are controlled to inflate to a specific pressure level and subsequently deflate.
Clause 11. The apparatus of clause 10, wherein the inflatable elements are inflated and deflated in a predetermined order, specifically wherein adjacent inflatable elements are inflated one after the other.
Clause 12. The apparatus of any one of clauses 1 - 11, further comprising a pump and one or more fluid distribution elements, particularly valves, and optionally electrovalves, in fluid communication with one or more of the actuators.
Clause 13. The apparatus of any one of clauses 1 - 12, wherein the sensing unit further comprises at least one of a resistivity sensor, a salinity sensor, an ultrasonic sensor, and a sound amplifier.
Clause 14. The apparatus of any one of clauses 1 - 13, wherein the control unit is configured to control the actuators such that a cyclic actuation with a frequency in the range of up to 0.9 cycles per second, specifically 0.1 - 0.9 cycles per second is generated.
Clause 15. The apparatus of any one of clauses 1 - 14, wherein the control unit is configured to operate the actuators such that a synchronized sequence of actuation is applied.
Clause 16. The apparatus of clause 15, wherein the sequence of actuation comprises a decreasing operating pressure in consecutive actuators from the abdominal surface adjacent to a base of the ascending colon towards the abdominal surface adjacent to the descending colon.
Clause 17. The apparatus of clause 15, wherein the sequence of actuation comprises an increasing operating pressure in consecutive actuators from the abdominal surface adjacent to a base of the ascending colon towards the abdominal surface adjacent to the descending colon.
Clause 18. The apparatus of any one of clauses 1 - 16, wherein the control unit is further configured to control the actuators such that the actuators are operated simultaneously.
Clause 19. A method for treating a patient, the method comprising:
   determining a physiological condition in at least a section of a colon of the patient; and
   a plurality of actuators applying a localized pressure against the abdominal wall in order to stimulate colon activity/evacuation; wherein
   the actuators apply the localized pressure at least partially based on the determined physiological condition.
Clause 20. The method of clause 19, wherein applying a localized pressure against the abdominal wall of the patient includes a cyclic actuation with a frequency in the range up to 0.9 cycles per second, specifically of 0.1 - 0.9 cycles per second.
Clause 21. The method according to clause 18 or 19, wherein the actuators include inflatable elements, and wherein the inflatable elements are controlled to inflate to a specific pressure level and subsequently deflate.
Clause 22. The method of clause 21, wherein the inflatable elements are inflated and deflated in a predetermined order, specifically wherein adjacent inflatable elements are inflated one after the other.
Clause 23. The method of any of clauses 18 - 22, wherein applying a localized pressure against the abdominal wall of the patient includes operating more than one actuator following a sequence of actuation.
Clause 24. The method of clause 23, wherein the sequence of actuation comprises operating the actuators such that an actuator operating pressure decreases in magnitude consecutively from the abdominal surface adjacent to a base of the ascending colon towards the abdominal surface adjacent to the descending colon.
Clause 25. The method of clause 23, wherein the sequence of actuation comprises operating the actuators such that an actuator operating pressure increases in magnitude consecutively from the abdominal surface adjacent to a base of the ascending colon towards the abdominal surface adjacent to the descending colon.
Clause 26. The method of any of clauses 23 - 25, wherein applying a localized pressure against the abdominal wall of the patient is performed by operating the actuators simultaneously.
Clause 27. The method of any one of clauses 18 - 26, wherein determining a physiological condition includes:
   activating one or more actuators that generate a localized pressure against the abdominal wall of the patient,
   measuring one or more parameters associated with a condition of a section of a colon of a patient, and
   processing the measured parameters to identify a physiological condition of the section of the colon.
Clause 28. The method of clause 27, wherein the measured parameters include pressure exerted by the actuators as a function of time, and wherein processing includes determining an operation time of the actuators until reaching a pressure threshold.
Clause 29. The method of clause 28, wherein processing includes computing a difference between a value of the time operation of the actuators to reach a predetermined pressure threshold with a reference value.
Clause 30. The method of clause 28, wherein processing includes estimating the frequency content of the recorded signal.
Clause 31. The method of any one of clauses 18 - 30, wherein identifying a physiological condition of the section of the colon includes:
   performing an ultrasonic scan of the abdomen of the patient.
Clause 32. The method of any one of clauses 18 - 31, wherein determining a physiological condition includes:
   performing a planar radiography of the abdomen of the patient.
Clause 33. The method of any one of clauses 18 - 32, wherein determining a physiological condition includes:
   performing a computerized tomography scan of the abdomen of the patient.
Clause 34. The method of any one of clauses 18 - 33, wherein determining a physiological condition includes:
   performing magnetic resonance imaging of the abdomen of the patient.
Clause 35. The method of any of clauses 18 - 34, wherein the method is computer-implemented, particularly wherein the method is executed by a control unit of the apparatus according to any of clauses 1 - 18.
Clause 36. A computer-implemented method for treating a patient, the method comprising:
   receiving data indicative of a physiological condition in at least a section of a colon of the patient from one or more sensing units;
   determining a physiological condition in at least a section of the colon; and
   controlling a plurality of actuators applying a localized pressure against the abdominal wall in order to stimulate colon activity/evacuation; wherein
   the actuators apply the localized pressure at least partially based on the determined physiological condition.
Clause 37. The computer-implemented method of clause 36, wherein determining a physiological condition includes:
   activating one or more actuators that generate a localized pressure against the abdominal wall of the patient,
   receiving data from sensing units measuring one or more parameters associated with a condition of a section of a colon of a patient, and
   processing the data or measured parameters to identify a physiological condition of the section of the colon.
Clause 38. The computer-implemented method of clause 37, wherein the measured parameters include pressure exerted by the actuators as a function of time, and wherein processing includes determining an operation time of the actuators until reaching a pressure threshold.
Clause 39. The computer-implemented method of clause 38, wherein the actuators include inflatable elements.
Clause 40. The computer-implemented method of clause 39, wherein the inflatable elements are controlled to inflate to a specific pressure level and subsequently deflate.
Clause 41. The computer-implemented method of clause 40, wherein the inflatable elements are inflated and deflated in a predetermined order, specifically wherein adjacent inflatable elements are inflated one after the other.
Clause 42. The computer-implemented method of any one of clauses 36-41, wherein the sensing unit further comprises at least one of a resistivity sensor, a salinity sensor, an ultrasonic sensor, and a sound amplifier.

This written description uses examples to disclose the teaching, including the preferred embodiments, and also to enable any person skilled in the art to practice the teaching, including making and using any devices or systems and performing any incorporated methods. The patentable scope is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims. Aspects from the various embodiments described, as well as other known equivalents for each such aspects, can be mixed and matched by one of ordinary skill in the art to construct additional embodiments and techniques in accordance with principles of this application. If reference signs related to drawings are placed in parentheses in a claim, they are solely for attempting to increase the intelligibility of the claim, and shall not be construed as limiting the scope of the claim.

## Claims

1. An apparatus configured to improve functioning of a patient's colon, comprising:
a support configured to be arranged at least partially around a waist of the patient and comprising one or more actuators configured to exert pressure against an abdominal wall of the patient;
a control unit configured to control the actuators; and
one or more sensing units configured to measure one or more parameters indicative of a condition of at least a section of a colon of a patient; wherein
the control unit is in communication with the sensing units, and is further configured to determine a physiological condition in the section of the colon of the patient and to control the actuators at least partially based on the physiological condition.

2. The apparatus of claim 1, wherein the sensing units are configured to measure an operating pressure of the actuators, and the control unit is configured to measure an operation time of the actuators until the operating pressure reaches a threshold.

3. The apparatus of claim 2, wherein the control unit is configured to determine a physiological condition in a section of the colon at least partially based on the measured operation time of the actuators and/or on a difference in operation time associated with different actuators.

4. The apparatus of any one of claims 1 - 3, wherein the control unit is configured to record a signal comprising at least one of the parameters measured by the sensing units as a function of time during operation of the actuators.

5. The apparatus of any of claims 1 - 4, wherein the control unit is configured to determine a physiological condition of a section of the colon at least partially based on a difference between a value of a parameter and a reference value.

6. The apparatus of any one of claims 1 - 5, comprising one or more actuators arranged such that in use they are located adjacent to an ascending section of the colon and one or more actuators arranged such that in use they are located adjacent to a descending section of the colon.

7. The apparatus of any one of claims 1 - 5, wherein the sensing unit comprises pressure sensors and wherein the actuators include inflatable elements.

8. The apparatus of claim 7, wherein the inflatable elements are controlled to inflate to a specific pressure level and subsequently deflate.

9. The apparatus of claim 8, wherein the inflatable elements are inflated and deflated in a predetermined order, specifically wherein adjacent inflatable elements are inflated one after the other.

10. The apparatus of any one of claims 1 - 9, wherein the sensing unit further comprises at least one of a resistivity sensor, a salinity sensor, an ultrasonic sensor, and a sound amplifier.

11. The apparatus of any one of claims 1 - 10, wherein the control unit is configured to control the actuators such that a cyclic actuation with a frequency in the range of up to 0.9 cycles per second, specifically 0.1 - 0.9 cycles per second is generated.

12. The apparatus of any one of claims 1 - 11, wherein the control unit is configured to operate the actuators such that a synchronized sequence of actuation is applied.

13. A computer-implemented method for treating a patient, the method comprising:
receiving data indicative of a physiological condition in at least a section of a colon of the patient from one or more sensing units;
determining a physiological condition in at least a section of the colon; and
controlling a plurality of actuators applying a localized pressure against the abdominal wall in order to stimulate colon activity/evacuation; wherein
the actuators apply the localized pressure at least partially based on the determined physiological condition.

14. The method of claim 13, wherein determining a physiological condition includes:
activating one or more actuators that generate a localized pressure against the abdominal wall of the patient,
measuring one or more parameters associated with a condition of a section of a colon of a patient, and
processing the measured parameters to identify a physiological condition of the section of the colon.

15. The method of claim 14, wherein the measured parameters include pressure exerted by the actuators as a function of time, and wherein processing includes determining an operation time of the actuators until reaching a pressure threshold.
